# EUROPEAN PATENT SPECIFICATION

(11) **EP 2 125 830 B1**
(45) Date of publication and mention of the grant of the patent: **26.01.2011**
(21) Application number: 08707692.3
(22) Date of filing: 13.02.2008
(51) Int. Cl.: C07D 498/04

(54) **NEW PROCESS FOR PREPARING 3-METHYL-4-PHENYLISOXAZOLO[3,4-D]PYRIDAZIN-7(6H)-ONE**
NEUES VERFAHREN FÜR DIE ZUBEREITUNG VON 3-METHYL-4-PHENYLISOXAZOLO[3,4-D]PYRIDAZIN-7(6H)-ON
NOUVEAU PROCÉDÉ DE PRÉPARATION DE 3-MÉTHYL-4-PHÉNYLISOXAZOLO[3,4-D]PYRIDAZIN-7(6H)-ONE

(30) Priority: 02.03.2007 ES 200700564
(43) Date of publication of application: 02.12.2009
(73) Proprietor: Almirall, S.A., 08022 Barcelona (ES)
(72) Inventor: MARCHUETA HEREU, Iolanda, E-08015 Barcelona (ES); SERRA MASIA, Xavier, E-08695 Bajá (Barcelona) (ES)
(74) Representative: Winkler, Andreas Fritz Ernst
(86) International application number: PCT/EP2008/001080
(87) International publication number: WO 2008/107064

(56) References cited:
- SPRIO, VINCENZO ET AL: "Nitrogen heterocycles. II. Hydrogenation of isoxazolo[3,4- d]pyridazin-7-ones, isoxazolo[3,4-d]pyridazin-4-ones, and isoxazolo[3,4-d]pyridazine-4,7-diones" ANNALI DI CHIMICA (ROME, ITALY), vol. 57, no. 7, 1967, pages 836-845, XP008085372 cited in the application

## Description

The present invention relates to a new process for preparing 3-methyl-4-phenylisoxazolo[3,4-d]pyridazin-7(6H)-one having the structure of formula (I):

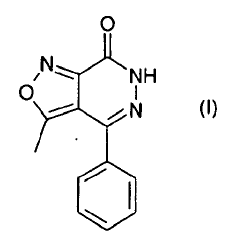

The compound of formula (I) is a useful intermediate in the preparation of some pyridazin-3(2H)-one derivatives which are selective inhibitors of phosphodiesterase 4 (PDE4) which have been described, for example, in the international patent applications numbers WO 03/097613 A1, WO 2004/058729 A1 and WO 2005/049581.

These patent applications also described a two step general method for the preparation of various isoxazolo[3,4-d]pyridazin-7(6H)-one derivatives carrying substituents in the positions 3 and 4 of the isoxazolo[3,4-d]pyridazin-7(6H)-one ring.

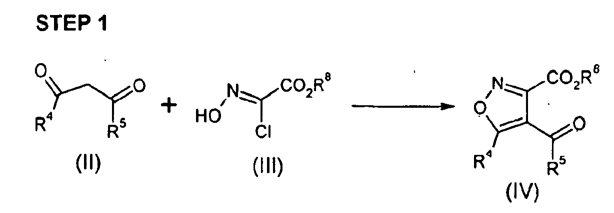

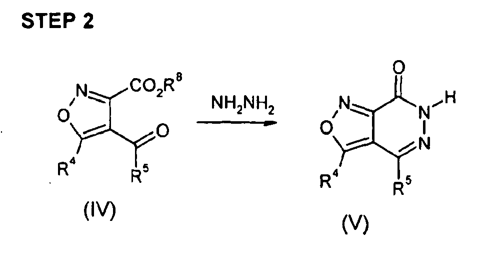

The general reaction conditions for the two steps of the above depicted method are also disclosed in the above mentioned applications. However the method is not exemplified for the synthesis of 3-methyl-4-phenylisoxazolo[3,4-d]pyridazin-7(6H)-one and consequently no yields are disclosed.

Step 1 of the above mentioned synthetic path is also described in the article G. Renzi, V. Dal Piaz et al, Gaz. Chim. Ital. 1968, 98, 656-666 and in V. Sprio, E.Aiello et al, Ann. Chim. 1967, 57, 836-845 both of which specifically disclose the synthesis of ethyl 4-benzoyl-5-methylisoxazole-3-carboxylate (Compound III, wherein R⁴=methyl, R⁵=phenyl and R^{B}=ethyl). The second article reports a yield of 40%.

Step 2 of the above mentioned synthetic path is also described in the article V. Sprio, E.Aiello et al, Ann. Chim. 1967, 57, 836-845 which specifically discloses the synthesis of 3-methyl-4-phenylisoxazolo[3,4-d]pyridazin-7(6H)-one (Compound IV wherein R⁴=methyl, and R⁵=phenyl). No yield is reported.

Whilst the above-mentioned two-step process is in some respects satisfactory, we have now devised an improved process allowing the manufacture of the compound of formula (I) with greater yields.

According to the present invention, there is provided a process for preparing the compound of formula (I): 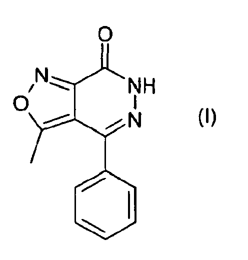 which process comprises the steps of:
a) reacting 1-phenylbutane-1,3-dione (IIa) 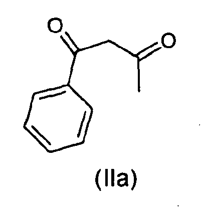 with ethyl 2-chloro-2-(hydroxyimino)acetate (IIIa) 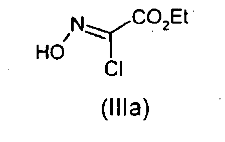
b) subsequently reacting the product from step (a) with hydrazine or an hydrate thereof to yield 3-methyl-4-phenylisoxazolo[3,4-d]pyridazin-7(6H)-one (I)
wherein the process is characterised by the fact that step (b) is carried out on the mixture resulting from step (a) without isolating ethyl 4-benzoyl-5-methylisoxazole-3-carboxylate (VIa) there from.

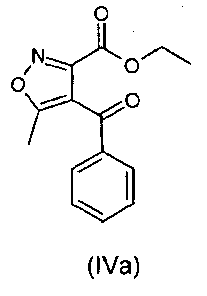

Thus, contrary to the previous methods, the process of the invention proceeds by directly treating with hydrazine (or its hydrate) the reaction mixture obtained in step (a) without isolating the reaction product there from.

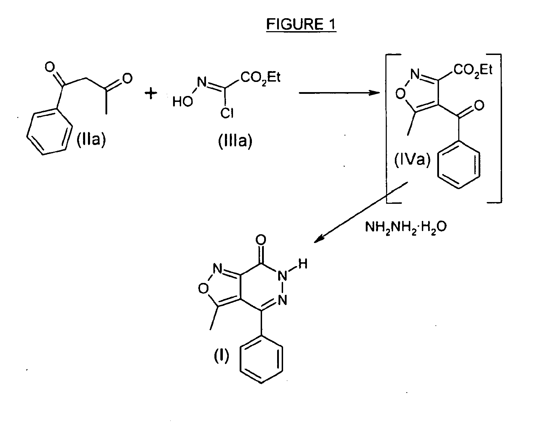

The above described reaction process is illustrated in Figure 1.

We have found that, surprisingly, the compound of formula (I) is obtained in a greater yield by carrying out the process in the above manner (without isolation of the compound (IVa)).

In an embodiment of the present invention the reactions are carried out using a C1-C3 alkanol, preferably methanol as a solvent.

In another embodiment of the present invention step a) is carried by addition of alkali alcoholate, preferably sodium methoxide followed by ethyl 2-chloro-2-(hydroxyimino)acetate.

In still another embodiment of the present invention the molar ratio of ethyl 2-chloro-2-(hydroxyimino)acetate to 1-phenylbutane-1,3-dione is comprised between 1,05 and 1,15.

In still another embodiment of the present invention the molar ratio of hydrazine hydrate to 1-phenylbutane-1,3-dione is comprised between 1,15 and 1,25.

The reactants 1-phenylbutane-1,3-dione (IIa), ethyl 2-chloro-2-(hydroxyimino)acetate (IIIa) and hydrazine hydrate used in the present invention are commercially available, for example from ABCR GmbH & CO. KG (P.O. Box 21 01 35, 76151 Karlsruhe, GERMANY), Aldrich Chemical Company, Inc (1001 West Saint Paul Avenue, Milwaukee, WI 53233, USA) or Fluka Chemie GmbH (Industriestrasse 25, P.O. Box 260, CH-9471 Buchs, SWITZERLAND).

The preferred conditions for the process of step (a) are the following:
Under an inert atmosphere, preferably under nitrogen atmosphere, 1 mol of 1-phenylbutane-1,3-dione is suspended in 300-350 ml of a C1-C3 alkanol, preferably methanol; and the suspension is cooled to 10-15°C. 190-200 g of MeONa 30% (methanolic solution) per mol of 1-phenylbutane-1,3-dione are added dropwise over 20-40 min at 10-15°C and the addition funnel is washed with 10-20 mL of methanol. The reaction mixture is stirred at 10-15°C for about 20-40 min and then cooled to 0-5°C. A solution of 166,7 g (1,1 mol) ethyl 2-chloro-2-(hydroxyimino)acetate in 300-350 mL of methanol are added dropwise over 50-70 min to the reaction mixture at 0-5°C and the addition funnel is washed with 10-20 mL of methanol.

The preferred conditions for the process of step (b) are the following:
The reaction mixture from step a) is warmed to 20-25°C and stirred for 100-150 min. Then 1,2 mol of NH₂NH₂.H₂O are added dropwise over 10-20 min at 35-45°C, the addition funnel is washed with 10-20 mL MeOH and the reaction mixture is stirred at 35-45°C for 220-260 min then cooled to 20-25°C. Finally 480-500 mL of water are added and the reaction mixture is stirred for 50-70 min at 20-25°C then 20-40 min at 0-5°C. The product is isolated by filtration then washed with 2x320 mL of cold MeOH/H₂O 1:1 and vacuum dried at 40-45°C overnight.

The method of synthesis described in the present invention will be further illustrated by the following examples. The examples are given by the way of illustration only and are not to be construed as limiting.

The structures of the prepared compounds were confirmed by ¹H-NMR and MS. NMR were recorded using a Varian Gemini-200 NMR spectrometer operating at frequency of 200 or 300 MHz. Tetramethyl silane was used as a reference and samples were solved in deuterated dimethylsulphoxide (DMSO-d₆) or deuterated chloroform (CDCl₃).

Their purity was determined by HPLC, in Alliance 2795 Waters instrument equipped with diode array detector (DAD) and ZMD or ZQ mass detector (electrospray ionization). HPLC method used a Symmetry C18 column (3.5 µm, 21x100 mm) and mobile phase was composed by two phases: Phase A: Buffered (Formic acid/ammonia) aqueous solution at pH: 3. Phase B: 50.50 mixture acetonitrile/methanol with ammonia formiate. Gradient was from 0% to 95% of phase B in 10 minutes.

Preparative HPLC-MS experiments were performed on a Gilson instrument equipped with a binary pump (Gilson piston pump 321); a vacuum degasser (Gilson 864); an injector-fraction collector (Gilson liquid handler 215); two injection modules, analytical and preparative (Gilson 819); a valve (Gilson Valvemate 7000); a 1/1000 splitter (Acurate by LC Packings); a make-up pump (Gilson 307); a diode array detector (Gilson 170) and a MS detector (a Thermoquest Finnigan aQa, a quadrupole mass spectrometer with ES and APCI ionisation modes). The HPLC-MS instrument was controlled by an IBM PC.

### EXAMPLES

### EXAMPLE 1

20g (0,123 mol) of 1-phenylbutane-1,3-dione and 40mL methanol are introduced under nitrogen atmosphere, into a four-necked flask fitted with mechanical stirrer and addition funnel and the suspension is cooled down to 10-15°C. 24.4g of a 30% solution of sodium methoxide in methanol are added dropwise over 30min at 10-15°C. The addition funnel is washed with 2mL of methanol.

The reaction mixture is stirred at 10-15°C for about 30min and then cooled to 0-5°C. A solution of 20.5g (0,135 mol) of ethyl-2-chloro-2-(hydroxyimino)acetate in 40mL MeOH is added dropwise over 1h to the reaction mixture at 0-5°C, the addition funnel is washed with 2mL methanol.

The reaction mixture is then warmed to 20-25°C and stirred for 2h. Without any isolation or purification step. 7.4g (0.148 mol) NH₂NH₂.H₂O are then added dropwise over 15min at 40°C, the addition funnel is washed with 2mL of methanol and the reaction mixture is stirred at 40°C for 4h then cooled to 20-25°C.

Finally 60mL of water are added and the reaction mixture is stirred for 1h at 20-25°C then 30min at 0-5°C.

The resulting mixture is filtered, the resulting solid is washed with 2x40mL cold MeOH/H₂O 1:1 and vacuum dried at 40°C overnight. Yielding 22,5 g (0,099 mol) of 3-methyl-4-phenylisoxazolo[3,4-d]pyridazin-7(6H)-one. (yield = 80,5%)

### COMPARATIVE EXAMPLE 2

162,19 g of 1-phenylbutane-1,3-dione (1 mol) are introduced under nitrogen atmosphere, into a four-necked flask fitted with mechanical stirrer and addition funnel and the suspension is cooled down to 10-15°C. 198 g of a 30% sodium methoxide / methanolic solution are added dropwise over 30min at 10-15°C. The addition funnel is washed with 2mL of methanol.

The reaction mixture is stirred at 10-15°C for about 30min and then cooled to 0-5°C. A solution of 166,25 g (1,10 mol) of ethyl-2-chloro-2-(hydroxyimino)acetate in 400mL MeOH is added dropwise over 1h to the reaction mixture at 0-5°C, the addition funnel is washed with 20mL methanol.

The final mixture was stirred at room temperature overnight. The crude was worked up and 262,86 g of an oil were isolated. The content of ethyl 4-benzoyl-5-methylisoxazole-3-carboxylate in the oil was determined to be 86% by CG-EL. This represents a yield of 87,1% (expressed as pure product).

### COMPARATIVE EXAMPLE 3

The 262,86 g of impure oil from Comparative example 2 were dissolved in 550 ml of methanol, warmed to 20-25°C and stirred for 2h. 78,12 g (1,56 mol) of NH₂NH₂.H₂O are then added dropwise over 15min at 40°C, the addition funnel is washed with 20mL of methanol and the reaction mixture is stirred at 40°C for 4h then cooled to 20-25°C.

Finally 480mL of water are added and the reaction mixture is stirred for 1h at 20-25°C then 30min at 0-5°C.

The resulting mixture is filtered, the resulting solid is washed with 2x40mL cold MeOH/H₂O 1:1 and vacuum dried at 40°C overnight. Yielding 168,2 g (0,740 mol) of 3-methyl-4-phenylisoxazolo[3,4-d]pyridazin-7(6H).one, (yield = 84,9%)
HPLC-MS(+/-)= 228
MS-FIA= 228

After cooling with an ice bath, a precipitate was formed and collected by filtration and washed with some pre-cooled alcoholic solvents

The combined yield of comparative examples 2 and 3 representing the yield of manufacturing 3-methyl-4-phenylisoxazolo[3,4-d]pyridazin-7(6H)-one in a two step reacting with isolation of the intermediate ethyl 4-benzoyl-5-methylisoxazole-3-carboxylate is 74,0%. (87,1% X 84,9%).

This yield is significantly lower that the yield obtained when the manufacturing process when it is carried out in the form of a one-pot reaction as claimed in the present invention and exemplified in Example 1.

## Claims

1. A process for preparing 3-methyl-4-phenylisoxazolo[3,4-d]pyridazin-7(6H)-one which process comprises the steps of:
a) reacting 1-phenylbutane-1,3-dione with ethyl 2-chloro-2-(hydroxyimino)acetate
b) subsequently reacting the product from step (a) with hydrazine or an hydrate thereof to yield 3-methyl-4-phenylisoxazolo[3,4-d]pyridazin-7(6H)-one
wherein the process is **characterised by** the fact that step (b) is carried out on the mixture resulting from step (a) without isolating ethyl 4-benzoyl-5-methylisoxazole-3-carboxylate there from.

2. A process according to claim 1 **characterized in that** the reactions are carried out using a C1-C3 alkanol as a solvent.

3. A process according to claim 2 **characterized in that** the alkanol is methanol.

4. A process according to any preceding claim **characterized in that** step a) is carried by addition of alkali alcoholate followed by ethyl 2-chloro-2-(hydroxyimino)acetate.

5. A process according to claim 4 wherein the alkali alcoholate is sodium methoxide.

6. A process according to any preceding claim wherein the molar ratio of ethyl 2-chloro-2-(hydroxyimino)acetate to 1-phenylbutane-1,3-dione is comprised between 1,05 and 1,15.

7. A process according to any preceding claim wherein the molar ratio of hydrazine hydrate to 1-phenylbutane-1,3-dione is comprised between 1,15 and 1,25.

## Patentansprüche

1. Verfahren zur Herstellung von 3-Methyl-4-phenylisoxazolo[3,4-d]pyridazin-7(6H)-on, wobei das Verfahren die Schritte umfasst:
a) Umsetzen von 1-Phenylbutan-1,3-dion mit Ethyl-2-chlor-2-(hydroxyimino)acetat
b) anschließend Umsetzen des Produktes aus Schritt (a) mit Hydrazin oder einem Hydrat davon, um 3-Methyl-4-phenylisoxazolo[3,4-d]pyridazin-7(6H)-on zu liefern
wobei das Verfahren **dadurch gekennzeichnet ist, dass** Schritt (b) an der Mischung durchgeführt wird, die aus Schritt (a) resultiert, ohne Ethyl-4-benzoyl-5-methylisoxazol-3-carboxylat daraus zu isolieren.

2. Verfahren nach Anspruch 1, **dadurch gekennzeichnet, dass** die Reaktionen unter Verwendung eines C1-C3-Alkanols als einem Lösemittel durchgeführt werden.

3. Verfahren nach Anspruch 2, **dadurch gekennzeichnet, dass** das Alkanol Methanol ist.

4. Verfahren nach einem vorangehenden Anspruch, **dadurch gekennzeichnet, dass** Schritt a) durchgeführt wird durch Zugabe von Alkalialkoholat, gefolgt von Ethyl-2-chlor-2-(hydroxyimino)acetat.

5. Verfahren nach Anspruch 4, wobei das Alkalialkoholat Natriummethoxid ist.

6. Verfahren nach einem vorangehenden Anspruch, wobei das Molverhältnis von Ethyl-2-chlor-2-(hydroxyimino)acetat zu 1-Phenylbutan-1,3-dion zwischen 1,05 und 1,15 liegt.

7. Verfahren nach einem vorangehenden Anspruch, wobei das Molverhältnis von Hydrazin-Hydrat zu 1-Phenylbutan-1,3-dion zwischen 1,15 und 1,25 liegt.

## Revendications

1. Procédé de préparation de 3-méthyl-4-phényl-isoxazolo[3,4-d]pyridazin-7-(6H)-one, ledit procédé comprenant les étapes suivantes :
a) réaction d'une 1-phénylbutane-1,3-dione avec un 2-chloro-2-(hydroxyimino)acétate d'éthyle
b) réaction ultérieure du produit de l'étape (a) avec une hydrazine ou un hydrate de celle-ci pour obtenir une 3-méthyl-4-phénylisoxazolo[3,4-d]pyridazin-7-(6H)-one
dans lequel le procédé est **caractérisé par le fait que** l'étape (b) est mise en oeuvre sur le mélange obtenu à l'étape (a) sans isolation du 4-benzoyl-5-méthylisoxazole-3-carboxylate d'éthyle à partir de celui-ci.

2. Procédé selon la revendication 1 **caractérisé en ce que** les réactions sont mises en oeuvre en utilisant un alcanol en C₁-C₃ à titre de solvant.

3. Procédé selon la revendication 2 **caractérisé en ce que** l'alcanol est le méthanol.

4. Procédé selon l'une quelconque des revendications précédentes **caractérisé en ce que** l'étape a) est mise en oeuvre par ajout d'un alcoolate alcalin, suivi de celui du 2-chloro-2-(hydroxyimino)acétate d'éthyle.

5. Procédé selon la revendication 4, dans lequel l'alcoolate alcalin est le méthoxyde de sodium.

6. Procédé selon l'une quelconque des revendications précédentes, dans lequel le rapport molaire du 2-chloro-2-(hydroxyimino)acétate d'éthyle à la 1-phénylbutane-1,3-dione est compris entre 1,05 et 1,15.

7. Procédé selon l'une quelconque des revendications précédentes, dans lequel le rapport molaire de l'hydrate d'hydrazine à la 1-phénylbutane-1,3-dione est compris entre 1,15 et 1,25.
